# EUROPEAN PATENT SPECIFICATION

(11) **EP 3 443 946 B1**
(45) Date of publication and mention of the grant of the patent: **30.12.2020**
(21) Application number: 17801955.0
(22) Date of filing: 09.03.2017
(51) Int. Cl.: A61H 39/06

(54) **MOXIBUSTION BOX**
MOXIBUSTION-BOX
BOÎTE DE MOXIBUSTION

(30) Priority: 23.05.2016 CN 201610340456
(43) Date of publication of application: 20.02.2019
(73) Proprietor: Toward Industrial Co. Ltd., Shanghai 201202 (CN)
(72) Inventor: SHIH, Shanwei, New Taipei City (CN)
(74) Representative: Gong, Jinping
(86) International application number: PCT/CN2017/076078
(87) International publication number: WO 2017/202105

(56) References cited:
- CN-A- 101 658 465
- CN-A- 104 248 510
- CN-A- 104 940 017
- CN-U- 201 676 159
- CN-U- 203 935 407
- CN-U- 204 972 225
- CN-U- 204 972 225
- CN-U- 205 163 620
- CN-U- 205 832 151
- CN-Y- 2 494 210
- CN-Y- 2 933 398
- CN-Y- 201 029 932
- JP-A- 2001 286 532
- JP-U- H0 353 239

## Description

### FIELD OF INVENTION

This invention relates to a box, more specifically, a moxibustion box.

### BACKGROUND OF THE INVENTION

In the study of Chinese medicine, moxibustion has many uses, such as tonifying qi, aiding yang, invigorating the spleen and kidney, replenishing the energy in human body, and resist aging. Long-term effects of moxibustion on the appropriate acupoints can even balance the yin and yang of the human body, nourish the spleen and stomach, fortify our center energy and qi, strengthen the body, avoid illness, and extend lifespan. Since the past thousands of years, the method of moxibustion treatment is widely used across China. Moxibustion starts by rolling dried moxa leaves into balls or rolls. Then light it to stimulate the acupoints with heat and the properties of moxa (Artemisiae Argyi) leaves. By stimulating the acupoints, the qi in the meridian channels of the human body can be replenished or transferred to better combat sickness and disease.

Current moxibustion treatments place moxa balls directly on the skin or simply uses a slice of ginger to insulate some of the heat. Many physicians also use moxa rolls and, after lighting the rolls, hold them above acupoints.

There is also the method of placing moxa rolls into moxibustion boxes. However, this method does not allow altering the distance between the burning moxa roll and the skin, thus unable to customize the treatment according to the needs and conditions of different patients. This lowers the quality of moxibustion treatment.

In current treatments, addendums such as ginger are often placed directly between the moxa and the skin to increase the increase the effectiveness of the treatment. However, this method is unstable and the moxa, along with the addendums, falls easily when the patient moves, disrupting the treatment process and effect.

Furthermore, in the current technology, even if the moxibustion tools have ways to change the distance with the skin or a window to air out the excessive heat, once the temperature exceed the regulation ability of the tools, it can cause burning , preventing the wound from properly sealing, and possibly amputation on patients with diabetes. It can cause even greater harm to disoriented patients or those in a coma.
Document CN2494210Y discloses a moxibustion box, comprising a casing, wherein a top portion and a bottom portion of the casing are respectively provided with an opening, a metal mesh sleeve, wherein the length of the sleeve is shorter than the length of the casing, an opening is provided on a top portion of the sleeve, and the rest of the sleeve has a mesh structure and the sleeve has a U-shaped cross-section; the shape of the inner wall of the sleeve corresponds to the shape used for moxa rolls and the outer wall of the sleeve corresponds to the shape of the casing; the top portion of the sleeve is provided with a protruding rim, wherein when the sleeve is inserted into the casing through the top opening of the casing, the protruding rim forms an interference fit with an edge of the top opening.

### DESCRIPTION OF THE INVENTION

The embodiment of this invention provides a type of moxibustion box that can solve the current problem of the unstableness of addendums. It also enables the usage of different types of herbs and medications, thus widening the treatment possibilities of moxibustion.

The feature of this invention consists of:
- A casing, wherein a top portion and a bottom portion of the casing are respectively provided with an opening, a horizontal slot is provided at the bottom portion.
- A metal mesh sleeve, wherein the length of the sleeve is shorter than the length of the casing, an opening is provided on a top portion of the sleeve, and the rest of the sleeve has a mesh structure. The sleeve has a U-shaped cross-section. The shape of an inner wall of the sleeve corresponds to the shape used for moxa rolls and the outer wall of the sleeve corresponds with the shape of the casing. The top portion of the sleeve is provided with a protruding rim. When the sleeve is inserted into the casing through the top opening of the casing, the protruding rim forms an interference fit with an edge of the top opening.
- A replaceable herbal container that can be slide through the horizontal slot provided at the bottom-side portion of the casing, which allows convenient replacement.
- The replaceable herbal container mentioned has a mesh structure, which allows the heat of the moxibustion to not only heat the herbs or tablets but also penetrate them and reach the acupoint.
- The replaceable herbal container mentioned above is used to install herbs or tablets.
- The side of the casing has an opening.
- The bottom ring has small pillars.
- Under the mentioned bottom ring mounts a ring of double-sided tape.
- Another replaceable herbal container can be round and can be installed upward into the bottom of the casing. Under the round herbal container mounts a ring of double-sided tape.
- Under the bottom part mentioned is installed with a temperature sensor assembly to monitor the temperature and emit an over-heat warning.

According to the embodiment of the invention, through the convenience of the replaceable herbal container, this invention can increase the number of therapeutic effects, in contrary to the limited effects of the original technique with only moxa and ginger. The invention provides a large number of therapeutic options by using different herbs for different acupoints, introducing novel changes to the practice of traditional Chinese medicine.

### DESCRIPTION OF THE DRAWINGS

FIG. 1 shows a front view of the Moxibustion Box;
FIG. 2 shows a side view of the Moxibustion Box;
FIG. 3 shows a cross-section view of the Moxibustion Box;
FIG. 4 shows a bottom view of the Moxibustion Box;
FIG. 5 shows a top view of the Moxibustion Box;
FIG. 6 shows the metal mesh sleeve;
FIG. 7 shows the combination of the casing and the metal sleeve;
FIG. 8 shows the replaceable herbal container;
FIG. 9 shows a cross-section view of the temperature sensor assembly or the wireless temperature sensor assembly installed to the bottom of the casing.
FIG. 10A and FIG. 10B shows another round replaceable herbal container;
FIG. 11 shows a cross-section view of the round replaceable herbal container installing upwards into the bottom of the casing.

### MODE OF CARRYING OUT THE INVENTION

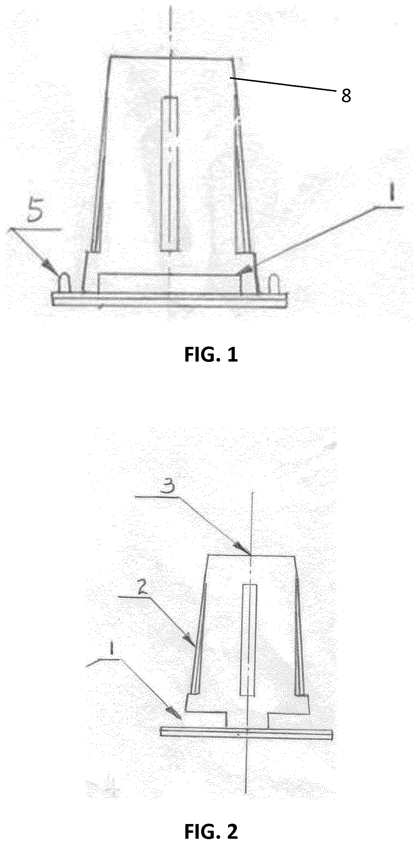

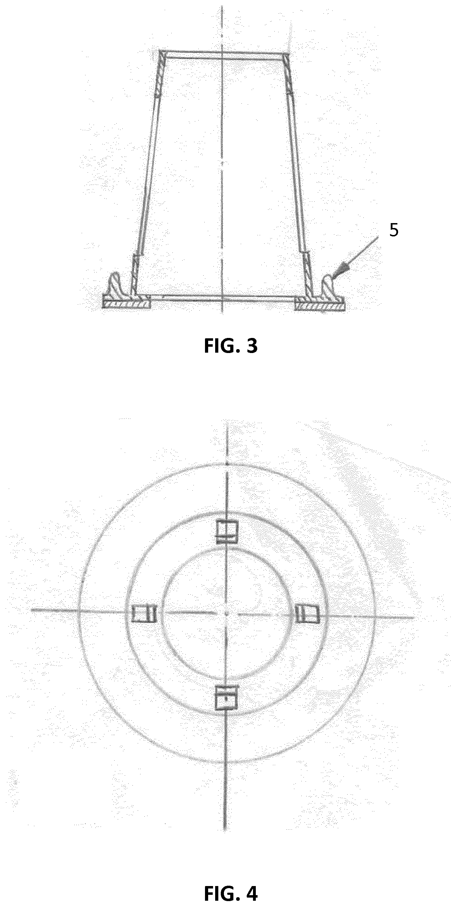

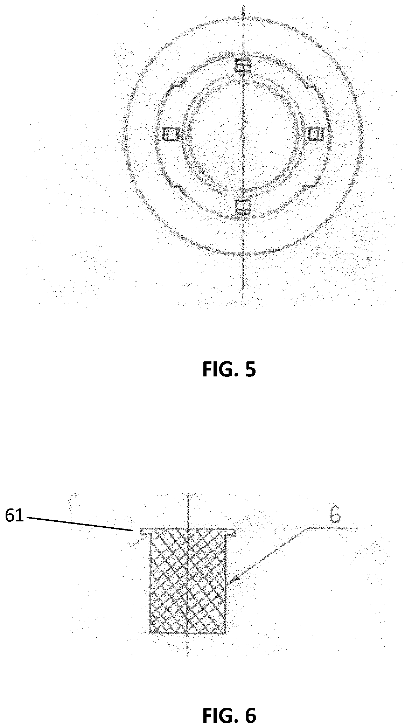

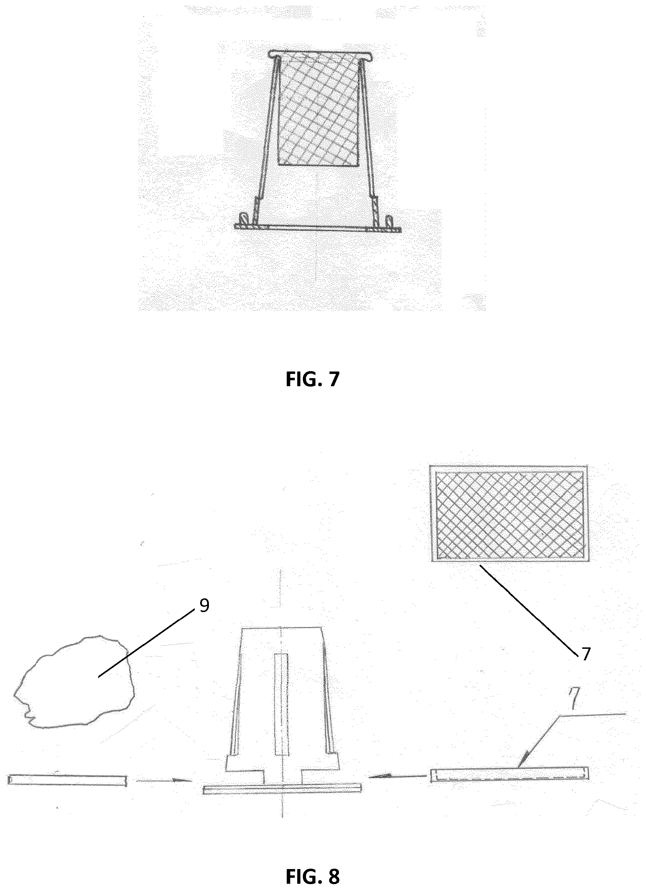

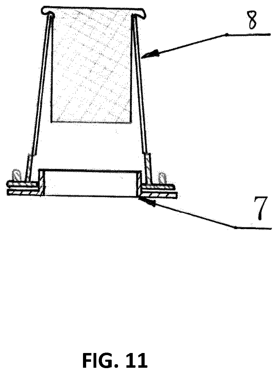

To better allow specialists within this field to understand and utilize the present invention, it will now be described by referencing the appended figures representing preferred embodiments. As FIG 1 to 11 show, the embodiment of this invention provides a type of moxibustion box, which consists of: a casing (8), wherein a top portion and a bottom portion of the casing are respectively provided with an opening,
and a slot (1) is provided at the bottom portion; a metal mesh sleeve (6), wherein the length of the sleeve is shorter than the length of the casing, an opening is provided on a top portion of the sleeve, and the rest of the sleeve has a mesh structure. The sleeve has a U-shaped cross-section. The shape of an inner wall of the sleeve corresponds to a shape used in moxibustion for moxa rolls and the outer wall of the sleeve corresponds with the shape of the casing. A top portion of the sleeve is provided with a protruding rim (61). When the sleeve is inserted into the casing (8) through the top opening of the casing, the protruding rim (61) forms an interference fit with an edge of the top opening. After the mentioned sleeve is installed into the casing (8) from the top, when the moxa roll is lit and inserted into the moxibustion box, the ashes won't fall through and burn the skin; the mentioned moxibustion box sleeve can be designed to have different thickness or clip to correspond to the different size and diameter of the moxa roll, thus able to fix the moxa roll or moxa ball in place.

The replaceable herbal container (7) can be slid through the horizontal slot provided at the bottom-side portion of the casing, which allows convenient replacement of different herbs or tablets when the moxibustion box is in use. The mentioned replaceable herbal container (7) is a mesh structure, which allows the heat of the moxibustion to not only heat the herbs or tablets but also penetrate them and reach the acupoint. The replaceable herbal container (7) mentioned is used to install herbs or tablets. The shape of the replaceable herbal container (7) can be different, the size of the herbs or tablet needs to be smaller than the size of the herbal container (7) in order for them to fit in. One could also modify the herbs or tablets to the same size as the replaceable herbal container so that they can be inserted directly into the slot. Just like the replaceable herbal container (7), the round replaceable herbal container also allows convenient replacement of different herbs or tablets when the moxibustion box is in use and is installed upwards into the bottom of the casing. Its bottom also has a mesh structure, which allows the heat of the moxibustion to not only heat the herbs or tablets but also penetrate. The components of the tablet can be synthetic preparations that assist the moxibustion curative effect, it can also be traditional plant-based slices such as ginger slices. The tablet can also be a box-packed powder and can also be a block-shaped formulation or a paste-shaped formulation to assist the curative effect in moxibustion. The components can be from traditional Chinese medicine, western medicine or a combination of both. This can break through the limitation of the traditional Chinese medicine method of only using moxa and ginger slices. Using different herbs or combining them into tablets, the heat of the moxibustion can penetrate the skin and reach the acupoints in the body, stimulating the meridian channels, and achieve the aim of treating diseases.

Additionally, the combination of the replaceable herbal container (7) and the casing (8) can be in other forms. For example, under the casing (8) is a groove, on the surface of the groove is a retractable protrusion. When the replaceable tablet container is placed in the groove, the protrusion will block the replaceable tablet container from falling out so that the replaceable tablet container and the casing form an integrated structure.

The Chinese medicines used in the tablets include extracts of Dang Gui (Angelica sinensis), Dan Shen (Salvia miltiorrhiza), Huang Qi (Astragalus membranaceus), and Chuan Xiong (Ligusticum striatum). This is because Dang Gui (Angelica sinensis) can enrich blood and tonify qi in the human body. Dan Shen (Salvia miltiorrhiza) can expand blood vessels. Huang Qi (Astragalus membranaceus) can also tonify qi. Apart from these herbs having the physical effect of acupuncture, the pharmacological effects include strengthening the body and treating diseases. Thus, they can improve the curative effect of moxibustion treatment.

The Western medicines used in the tablets include vitamin B1, B12, C, K, Ephedrine, antibiotic... etc.

To create the tablets, auxiliary gels (such as carbomer, gelatin, albumin, alginate, chitosan...etc.) can be used for processing the herbs and medicines. Various different processing technologies can be adopted. For example, extracting the raw materials, atomizing to prepare powder, extruding, or carrying out low-temperature drying and the like to prepare the tablets, so as to produce tablets of different types, and, with supporting tools, facilitate the usage of said tablets.

There are openings provided at the sides of the casing (8) for air circulation to aid the moxibustion burning. The casing can be of many different shapes and size, such as square or circular.

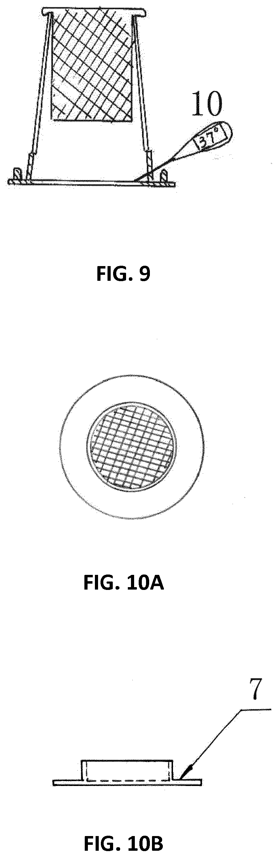

Additionally, as shown in FIG. 9, a temperature sensor assembly (10) can be installed at the bottom. The temperature sensor assembly (10) consists of a temperature sensor unit, a settings unit, and an alarm unit. When the temperature measured by the temperature sensor unit is higher than the predetermined value, the alarm unit will send out an alarm. It can also use a wireless transmitter unit to display the measured temperature to a monitor. When the temperature is higher than the predetermined value it will send out an alarm. For example, the predetermined value for a normal patient is 43 degrees Celsius, the predetermined value for a patient with diabetes will be 41 degrees Celsius at most. This is according to the studies of the earliest scholars that researched burns, Henriques and Moritzu. When the temperature of biological tissues exceeds 44 degrees Celsius it will cause thermal damage. Diabetes patients' thermal conductivity(W/(m · K) of the epidermal layer, dermis layer, and subcutaneous tissue is lower than that of a normal patient due to blood sugar. Thus their predetermined value can be customized based on each person. Once the temperature exceeds the predetermined value, the temperature sensor assembly will set off an alarm to remind the specialist to immediately remove the heat source.

Under the bottom ring of the mentioned casing is a ring of double-sided tape that can be secured on the skin. The small pillars on the bottom ring can be fixed with strings such as rubber bands to be tied on the patient's body. Another method is to use buckles to secure ribbons on the pillars to be tied on patient's limbs. This way, the addendums such as tablets or ginger in the replaceable tablet container can be used on tilted or upside down positions. The number and shape of the pillars are not restricted.

A round replaceable herbal container can be installed upward into the bottom of the casing. Under the round herbal container mounts a ring of double-sided tape.

The embodiment of this invention provides a method of moxibustion treatment. In the current technology of simulating acupoints to achieve a curative effect, this invention solves the difficulty of keeping the addendums such as ginger in place due to the texture. Through the use of different herbs and medicines or the properties of different textures, it can increase the curative effect of specific needs. The shapes of tablets used in this invention can be solid or gelatin. The tablet can consist of Chinese medicine, Western medicine, a combination of both, or natural food ingredients such as ginger.

The embodiment of this invention provides a method of moxibustion treatment, improving the current moxibustion technology of only using flat skin surfaces to able to treat acupoints at tilted or upside down positions.

The tablet mentioned can increase the curative effect of specific needs through the use of different herbs and medicines or the properties of different textures. The shape of the mentioned tablets can be slice shaped, box-packed powder or extruded into slabs. It can be made up of Chinese medicine, Western medicine, a combination of both, or natural food ingredients such as ginger. This can break through the limitation of the traditional Chinese medicine method of only using moxa and ginger slices. Using different herbs, combining them into tablets, or paste formulation and placing them into the mesh structure of the herbal container, the heat of the moxibustion can treat through the skin.

According to the embodiment of this invention, the moxibustion treatment methods of the moxibustion box in this invention, by using various herbs and tablets, can increase the therapeutic effect and break through the limitation of current moxibustion technology of using flat surface skin and be able to treat vertical and upside down acupoints. It can also lower the disruption of moxibustion treatment on work and movements. The method it uses is fixing the small pillars on the bottom ring with strings such as rubber bands to be tied on the patient's body; using buckles to secure ribbons on the pillars to be tied on patient's limbs, or using the bottom double-sided tape to stick on the skin.

Additionally, this invention can increase the curative effect of moxibustion by choosing different tablet and acupoints according to different illness and the relations of meridian channels.

Furthermore, when creating different types of tablets for this invention, the classification and preservation of numerous types of tablets would not add to the hassle of the specialists due to its small size and light weight.
Moreover, this invention protects the safety of patients with conditions such as diabetes, who has lower heat sensitivity and whose burn wounds are more difficult to heal. It also protects the safety of disoriented patients or those in a coma, who could not properly react to heat sensation. Thus, this invention provides a replaceable herbal container and, through it, provides a new method of moxibustion. It can increase the curative effects. Especially, it provides a large number of therapeutic options by using different herbs for different acupoints, introducing novel changes to the practice of traditional Chinese medicine.

Although the invention is depicted by the embodiments, those of ordinary skill in this field knows that many modifications and variations can be made to the present invention without departing from the invention. The scope of the invention is defined by the appended claims.

## Claims

1. A moxibustion box, comprising
a casing, wherein a top portion and a bottom portion of the casing are respectively provided with an opening, a horizontal slot is provided at the bottom portion;
a metal mesh sleeve, wherein the length of the sleeve is shorter than the length of the casing, an opening is provided on a top portion of the sleeve, and the rest of the sleeve has a mesh structure and the sleeve has a U-shaped cross-section;
the shape of the inner wall of the sleeve corresponds to the shape used for moxa rolls and the outer wall of the sleeve corresponds to the shape of the casing;
the top portion of the sleeve is provided with a protruding rim, wherein when the sleeve is inserted into the casing through the top opening of the casing, the protruding rim forms an interference fit with an edge of the top opening;
a replaceable herbal container that can be slid through the horizontal slot provided at the bottom-side portion of the casing, which allows convenient replacement during treatment.

2. The moxibustion box according to claim 1, wherein the replaceable herbal container mentioned has a mesh structure, which allows the heat of the moxibustion to not only heat the herbs or tablets but also penetrate them.

3. The moxibustion box according to claim 1, wherein the replaceable herbal container mentioned above is suitable for installing herbs or tablets.

4. The moxibustion box according to claim 1, wherein the side of the mentioned casing has an opening.

5. The moxibustion box according to claim 1, wherein said bottom portion of the casing has a bottom ring, and wherein said bottom ring has small pillars.

6. The moxibustion box according to claim 1, wherein said bottom portion of the casing has a bottom ring, and wherein under said bottom ring mounts a ring of double-sided tape.

7. The moxibustion box according to claim 1, wherein another replaceable herbal container is round and can be installed upward into the bottom of the casing, wherein under the round herbal container a ring of double-sided tape is mounted.

8. The moxibustion box according to claim 1, wherein under the bottom part mentioned is installed with a temperature sensor assembly to monitor the temperature and emit an over-heat warning.

## Patentansprüche

1. Eine Moxibustionsbox, es zeichnet sich aus durch:
Die Schale ist am oberen und unteren Teil mit Öffnungen versehen, und unten ist ein horizontaler Schlitz vorgesehen;
Die Länge der Moxibustion-Metallgitterabdeckung ist kürzer als die Länge der Schale, der obere Teil ist mit einer Öffnung versehen, der andere Teil ist eine Maschenstruktur und der Abschnitt ist U-förmig.Die Form der Innenwand entspricht der Form der Moxibustion, und die Form der Außenwand entspricht der Form der Schale. Der obere Teil ist mit einem Flansch versehen, so dass der Flansch durch die Schale blockiert wird, wenn er in der oberen Öffnung der Schale verschachtelt ist. Der Umfang der oberen Öffnung;
Austauschbare Tablet-Box, die zum Austausch in den horizontalen Schlitz an der Unterseite des Gehäuses eingesetzt wird.

2. Moxibustionsbox nach Rechtsforderung 1, wobei die austauschbare Tablettenbox eine Maschenstruktur aufweist, so dass die Moxibustionswärme die Tablette erwärmen und dann in die Tablette eindringen kann.

3. Moxibustionsbox nach Rechtsforderung 1, wobei die austauschbare Tablettenbox zum Platzieren von Tabletten verwendet wird.

4. Moxibustionsbox nach Rechtsforderung 1, wobei die Seitenfläche des Gehäuses mit einer Öffnung versehen ist.

5. Moxibustionsbox nach Rechtsforderung 1, wobei sich über dem unteren Ring kleine Säulen befinden.

6. Moxibustionsbox nach Rechtsforderung 1, wobei der Boden mit einem ringförmigen doppelseitigen Klebeband versehen ist.

7. Moxibustionsbox nach Rechtsforderung 1, wobei die austauschbare Tablettenbox eine kreisförmige Form hat und verwendet wird, um vom Boden des Gehäuses nach oben eingeführt zu werden, und der Boden der kreisförmigen Tablettenbox mit einem ringförmigen doppelseitigen Klebstoff versehen ist.

8. Moxibustionsbox nach Rechtsforderung 1, wobei der Boden mit einer Temperatursensorkomponente versehen ist, um die Temperatur des Bodens zu erfassen und eine Übertemperaturwarnung auszugeben.

## Revendications

1. Une type de boîte à moxibustion, **caractérisée par**, y compris:
Un boîtier, dont la partie supérieure et la partie inférieure sont pourvues d'ouvertures, et dont le fond est pourvu d'une fenêtre latérale;
Grille métallique pour moxibustion, sa longueur est plus courte que celle de la boîtier, avec des ouvertures situées au-dessus, les autres parties étant en treillis et le profil en forme de U, la forme de la paroi intérieure correspond à celle de la moxibustion et la forme de la paroi extérieure à celle de la boîtier, sa partie supérieure est munie de bords saillants, de telle sorte que, lorsqu'ils sont ancrés dans l'ouverture supérieure de la boîtier, les bords saillants soient bloqués contre le périmètre de l'ouverture supérieure de la boîtier;
Boîtes de comprimés sont remplaçables, dont le fond du boîtier est insérée dans une fenêtre latérale pour permettre le remplissage.

2. Selon les boîtes à moxibustion décrites dans la *Revendication du droit 1,* sont **caractérisées par le fait que**, les boîtes de comprimés remplaçables décrites ci-dessus ont une structure réticulaire qui permet de chauffer les comprimés avant de les pénétrer.

3. Selon les boîtes à moxibustion décrites dans la *Revendication du droit 1,* sont **caractérisées par le fait que**, les boîtes de comprimés remplaçables décrites ci-dessus sont utilisées pour placer les comprimés.

4. Selon les boîtes à moxibustion décrites dans la *Revendication du droit 1,* sont **caractérisées par le fait que**, des ouvertures disposées sur les côtés de la boîtier décrite ci-dessus.

5. Selon les boîtes à moxibustion décrites dans la *Revendication du droit 1,* sont **caractérisées par le fait qu'**il y a de petites colonnes au-dessus de l'anneau au fond.

6. Selon les boîtes à moxibustion décrites dans la *Revendication du droit 1,* sont **caractérisées par le fait que**, le fond décrit ci-dessus est équipé d'une gomme doubleface annulaire.

7. Selon les boîtes à moxibustion décrites dans la *Revendication du droit 1,* sont **caractérisées par le fait que**, les boîtes de comprimés remplaçables décrites ci-dessus sont circulaires et sont destinées à être insérées vers le haut à partir du fond du boîtier, tandis que les boîtes de comprimés circulaires sont munies au fond d'une gomme doubleface annulaire.

8. Selon les boîtes à moxibustion décrites dans la *Revendication du droit 1,* sont **caractérisées par le fait qu'**il est équipé d'un ensemble de capteurs de température au fond décrit ci-dessus, permettant de détecter la température du fond et d'envoyer une alarme de sur-température.
